# EUROPEAN PATENT APPLICATION

(11) **EP 3 925 945 A1**
(43) Date of publication of application: **22.12.2021**
(21) Application number: 21188721.1
(22) Date of filing: 08.03.2017
(51) Int. Cl.: C07C 17/275, C07C 19/08, C07C 22/08, C07C 41/30, C07C 43/174, C07C 67/343, C07C 69/24, C07C 69/608, C07C 69/612, C07C 69/675, C07B 37/02, C07B 61/00, C07B 37/04, C07C 17/266

(54) **METHOD FOR PRODUCING FLUORINATED COMPOUND**

(30) Priority: 08.03.2016 JP 2016044901; 26.12.2016 JP 2016251305
(62) Divisional of application: 17763288.2
(71) Applicant: Daikin Industries, Ltd., Osaka-shi, Osaka 530-8323 (JP); University Public Corporation Osaka, Osaka-shi, Osaka 545-0051 (JP)
(72) Inventor: Matsuura, Makoto, Osaka, 530-8323 (JP); Yamamoto, Akinori, Osaka, 530-8323 (JP); Kishikawa, Yosuke, Osaka, 530-8323 (JP); Ryu, Ilhyong, Osaka, 599-8531 (JP); Fukuyama, Takahide, Osaka, 599-8531 (JP); Sumino, Shuhei, Osaka, 599-8531 (JP)
(74) Representative: Hoffmann Eitle

(57) **Abstract**

This invention solves the problem of providing an efficient, new method for producing a fluoromethylene-containing compound. The problem can be solved by a method for producing a compound represented by formula (1) or a ring-closed or ring-opened derivative of the compound, wherein R¹ represents an organic group, R^{x} represents hydrogen or fluorine, R^{2a}, R^{2b}, R^{2c}, and R^{2d} are the same or different, and each represents -Y-R²¹ or - N (-R²²)₂, or R^{2b} and R^{2c} may join together to form a bond, wherein Y represents a bond, oxygen, or sulfur, R²¹ represents hydrogen or an organic group, and R²², in each occurrence, is the same or different and represents hydrogen or an organic group; the method comprising step A of reacting a compound represented by formula (2), wherein X represents a leaving group, and other symbols are as defined above, with a compound represented by formula (3), wherein the symbols are as defined above, in the presence of a reducing agent under light irradiation.

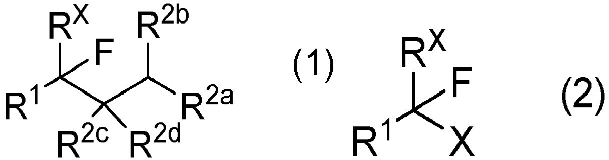

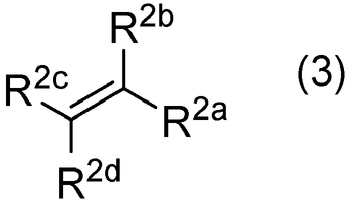

## Description

### Technical Field

The present invention relates to a method for producing a fluorine-containing compound, in particular to a method for producing a compound having a fluoromethylene group.

### Background Art

Since some physiological active substances *in vivo* are fluorinated methylene-containing compounds, applications of fluoromethylene-containing compounds to drugs etc. have been actively studied.

For example, methods for producing fluorine-containing methylene compounds, such as α-fluoromethylene compounds and **α-**difluoroaldol compounds, which are carbonyl compounds having at the α-position at least one substituent selected from the group consisting of fluorine atoms and perfluoro organic groups, are highly useful (Non-patent Literature 1 and 2).

As a method for producing a carbonyl compound having at the α-position at least one substituent selected from the group consisting of fluorine atoms and perfluoro organic groups, little has been reported so far on a method for producing a fluorine-containing carbonyl compound in which easily available trifluoromethyl ketone and a carbonyl compound are used as starting materials. An efficient, easy production method using such starting materials has been desired.

In Non-patent Literature 3, an α-difluoroaldol compound is obtained from trifluoromethyl acetone; however, five steps are required. Additionally, use of thiophenol as a reagent requires a highly toxic reagent such as mercury chloride to remove sulfur from the reaction system, which makes reaction operation complicated.

In Non-patent Literature 4, an α-difluoroaldol compound is obtained by reacting 2,2-difluoroenol silyl ether, which has been obtained by magnesium-related selective C-F bond cleavage in trifluoromethyl ketone, with benzaldehyde in the presence of chlorotrimethylsilane; however, such reaction requires three steps and also requires a massive amount of a reagent for the reaction substrate. Accordingly, there is a room for improvement from the viewpoint of yield etc.

Moreover, since all of the above methods produce inorganic salts as by-products, the step of removing the salts is required. Thus, in view of production costs, reaction efficiency, easiness, etc., an improved or completely new production method has been desired.

Non-patent Literature 5 discloses visible-light-mediated intramolecular addition of glycosyl halides to olefines that are substituted with electron-withdrawing groups, in which a compound containing no fluorine is used as a substrate, and amine, and Hantzsch ester as an auxiliary for the amine are used.

Non-patent Literature 6 discloses a method for producing a compound having a fluoromethylene group, the method using a compound containing no fluorine as a substrate.

### Citation List

### Non-patent Literature

NPL 1: John T. Welch et al., Tetrahedron, 1987, 43, 14, p.3123
NPL 2: Svante et al., J. Am. Chem. Soc., 1981, 103, p.4452
NPL 3: In Howa et al., Synthetic Communications, 1999, 29 (2), p.235
NPL 4: Amii et al., Chem. Commun., 1999, p.1323
NPL 5: Andrews et al., Angew. Chem. Int. Ed., 2010, 49, p.7274
NPL 6: Yu et al., Chem. Commun., 2014, 50, p.12884

### Summary of Invention

### Technical Problem

An object of the present invention is to provide a novel method for efficiently producing a compound having a fluoromethylene group.

### Solution to Problem

As a result of extensive research, the inventors found that the problem can be solved by the following method.

A method for producing a compound represented by the following formula (1) or a ring-closed or ring-opened derivative of the compound (sometimes referred to as "compound (1)" in the specification): 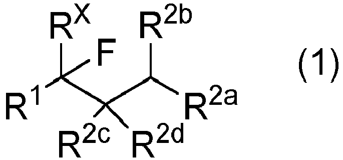 wherein R¹ represents an organic group,
R^{X} represents hydrogen or fluorine,
R^{2a}, R^{2b}, R^{2c}, and R^{2d} are the same or different, and each represents -Y-R²¹ or -N(-R²²)₂, or R^{2b} and R^{2c} may join together to form a bond,
wherein Y represents a bond, oxygen, or sulfur,
R²¹ represents hydrogen or an organic group, and
R²², in each occurrence, is the same or different and represents hydrogen or an organic group;
the method comprising step A of reacting a compound (sometimes referred to as "compound (2)" in the specification) represented by formula (2): 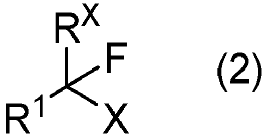 wherein X represents a leaving group, and other symbols are as defined above,
with a compound (sometimes referred to as "compound (3)" in the specification) represented by formula (3): 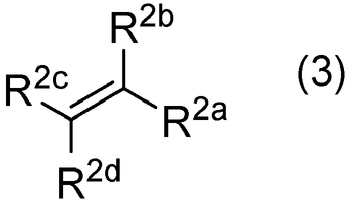 wherein the symbols are as defined above,
in the presence of a reducing agent under light irradiation.

The inventors thus accomplished the invention.

The present invention includes the following.

### Item 1.

A method for producing a compound represented by the following formula (1) or a ring-closed or ring-opened derivative of the compound: 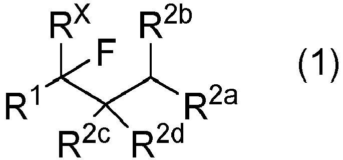 wherein R¹ represents an organic group,
R^{X} represents hydrogen or fluorine,
R^{2a}, R^{2b}, R^{2c}, and R^{2d} are the same or different, and each represents -Y-R²¹ or -N(-R²²)₂, or R^{2b} and R^{2c} may join together to form a bond, wherein Y represents a bond, oxygen, or sulfur,
R²¹ represents hydrogen or an organic group, and
R²², in each occurrence, is the same or different and represents hydrogen or an organic group;
the method comprising step A of reacting a compound represented by formula (2): 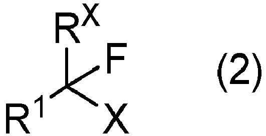 wherein X represents a leaving group, and other symbols are as defined above, with
a compound represented by formula (3): 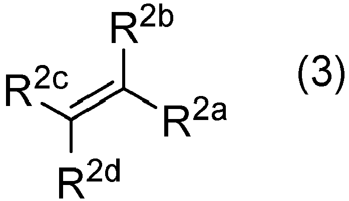 wherein the symbols are as defined above, in the presence of a reducing agent under light irradiation

### Item 2.

The method according to Item 1, wherein R¹ is alkyl, fluoroalkyl, alkoxycarbonyl, or an aromatic group.

### Item 3.

The method according to Item 1 or 2, wherein R^{2a} is alkyl or aryl, and R^{2b}, R^{2c}, and R^{2d} are each hydrogen.

### Item 4.

The method according to any one of Items 1 to 3, wherein X is bromine.

### Item 5.

The method according to any one of Items 1 to 4, wherein the reaction of step A is performed in the presence of a nitrogen-containing unsaturated heterocyclic compound having an N-H moiety.

### Item 6.

The method according to any one of Items 1 to 5, wherein the reducing agent is a compound represented by formula (4) : 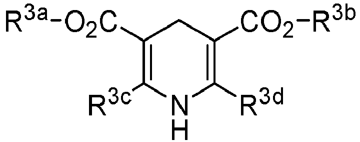 wherein R^{3a}, R^{3b}, R^{3c}, and R^{3d} are the same or different, and each represents alkyl.

### Item 7.

The method according to any one of Items 1 to 6, wherein the reaction of step A is performed in the presence of a catalyst.

### Item 8.

The method according to Item 7, wherein the catalyst is at least one member selected from the group consisting of transition metal complexes and organic dye compounds.

### Advantageous Effects of Invention

The present invention provides an efficient, novel method for producing a compound having a fluoromethylene group.

### Description of Embodiments

### Terms

The symbols and the abbreviations in this specification are to be interpreted as having the general meanings in the related technical field to which the present invention pertains, according to the context of this specification, unless otherwise specified.

In this specification, the terms "comprise" and "contain" encompasse the meanings of "consist essentially of" and "consist of."

The steps, treatments, or operations in this specification can be performed at room temperature unless otherwise specified.

In this specification, room temperature refers to a temperature in a range of 10 to 40°C.

In this specification, "Cn-m" (herein, n and m are each natural numbers) indicates that the carbon number is n or more and m or less, as conventionally used in the organic chemical field.

In this specification, the term "fluoromethylene" includes monofluoromethylene and difluoro methylene unless otherwise specified.

In this specification, unless otherwise specified, examples of "halogen atom" include fluorine, chlorine, bromine, and iodine.

In this specification, unless otherwise specified, the term "organic group" refers to a group containing at least one carbon atom as its constituent atom.

In this specification, unless otherwise specified, examples of "organic group" include hydrocarbon, cyano, carboxy, alkoxy, ester, ether, and acyl.

In this specification, unless otherwise specified, the term "hydrocarbon" refers to a group containing at least one carbon atom and at least one hydrogen atom as its constituent atoms.

In this specification, unless otherwise specified, examples of "hydrocarbon" include aliphatic hydrocarbon, aromatic hydrocarbon (aryl), and combinations thereof.

In this specification, unless otherwise specified, the term "aliphatic hydrocarbon" may be linear, branched, or cyclic aliphatic hydrocarbon, or a combination thereof.

In this specification, unless otherwise specified, the term "aliphatic hydrocarbon" may be saturated or unsaturated aliphatic hydrocarbon.

In this specification, unless otherwise specified, examples of "aliphatic hydrocarbon" includes alkyl, alkenyl, alkynyl, and cycloalkyl.

In this specification, unless otherwise specified, the term "alkyl" refers to linear or branched C₁₋₁₀ alkyl, such as methyl, ethyl, propyl, isopropyl, butyl, isobutyl, sec-butyl, tert-butyl, pentyl, isopentyl, neopentyl, and hexyl.

In this specification, the term "fluoroalkyl" refers to alkyl having at least one hydrogen atom replaced by a fluorine atom.

In this specification, the number of fluorine atoms in the "fluoroalkyl" may be one or more (e.g., 1 to 3, 1 to 6, 1 to 12, or 1 to the maximum replaceable number).

The term "fluoroalkyl" includes perfluoroalkyl. The term "perfluoroalkyl" refers to alkyl having all hydrogen atoms replaced by fluorine atoms.

In this specification, unless otherwise specified, the term "alkenyl" refers to linear or branched C₁₋₁₀ alkenyl, such as vinyl, 1-propenyl, isopropenyl, 2-methyl-1-propenyl, 1-butenyl, 2-butenyl, 3-butenyl, 2-ethyl-1-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, and 5-hexenyl.

In this specification, unless otherwise specified, the term "alkynyl" refers to linear or branched C₂₋₆ alkynyl, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, and 5-hexynyl.

In this specification, unless otherwise specified, the term "cycloalkyl" refers to C₃₋₁₀ cycloalkyl (preferably C₄₋₁₀ cycloalkyl), such as cyclopentyl, cyclohexyl, and cycloheptyl.

In this specification, unless otherwise specified, the term "alkoxy" refers to, for example, a group represented by RO-(wherein R represents alkyl).

In this specification, unless otherwise specified, the term "ester" refers to, for example, a group represented by formula RCO₂- (wherein R represents alkyl).

In this specification, unless otherwise specified, the term "ether" refers to a group having an ether bond (-O-), and examples of ether include polyether. Examples of polyether include groups represented by formula Rₐ-(O-R_{b})ₙ- (wherein Rₐ represents alkyl, R_{b}, in each occurrence, is the same or different and represents alkylene, and n is an integer of 1 or more). Alkylene is a divalent group formed by removing one hydrogen atom from an alkyl group.

In this specification, unless otherwise specified, the term "acyl" includes alkanoyl. In this specification, unless otherwise specified, the term "alkanoyl" refers to, for example, a group represented by RCO- (wherein R represents alkyl).

In this specification, unless otherwise specified, the term "aromatic group" includes aryl and heteroaryl.

In this specification, examples of "aryl" include C₆₋₁₀ aryl, such as phenyl and naphthyl.

In this specification, examples of "heteroaryl" include 5- to 14-membered (monocyclic, dicyclic, or tricyclic) heterocyclic groups containing, in addition to carbon, 1 to 4 heteroatoms selected from the group consisting of nitrogen, sulfur, and oxygen as an annular atom.

In this specification, examples of "heteroaryl" include
(1) monocyclic aromatic heterocyclic groups, such as furyl, thienyl, pyridyl, pyrimidinyl, pyridazinyl, pyrazinyl, pyrrolyl, imidazolyl, pyrazolyl, thiazolyl, isothiazolyl, oxazolyl, isoxazolyl, oxadiazolyl, thiadiazolyl, and triazolyl, tetrazolyl, and triazinyl; and
(2) polycyclic (e.g., dicyclic) aromatic heterocyclic groups, such as quinolyl, isoquinolyl, quinazolyl, quinoxalyl, benzofuryl, benzothienyl, benzoxazolyl, benzisoxazolyl, benzothiazolyl, benzimidazolyl, benzotriazolyl, indolyl, indazolyl, pyrrolo pyrazinyl, imidazo pyridinyl, imidazo pyrazinyl, imidazo thiazolyl pyrazolo pyridinyl, pyrazolo thienyl, and pyrazolo thoriadinyl.

### Production Method

The method for producing a compound represented by the following formula (1) or a ring-closed or ring-opened derivative of the compound: 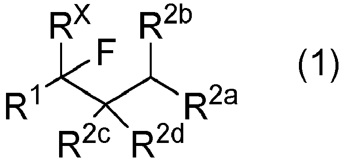 wherein R¹ represents an organic group,
R^{X} represents hydrogen or fluorine,
R^{2a}, R^{2b}, R^{2c}, and R^{2d} are the same or different, and each represents -Y-R²¹ or -N(-R²²)₂, or R^{2b} and R^{2c} may join together to form a bond,
wherein Y represents a bond, oxygen, or sulfur,
R²¹ represents hydrogen or an organic group, and R²², in each occurrence, is the same or different and represents hydrogen or an organic group,
comprises step A of reacting a compound represented by formula (2) : 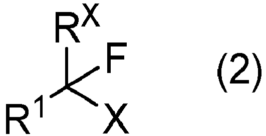
wherein X represents a leaving group, and other symbols are as defined above, with a compound represented by formula (3): 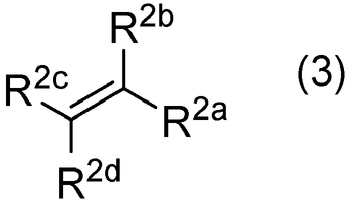 wherein the symbols are as defined above, in the presence of a reducing agent under light irradiation.

The symbols in the chemical formulae above are explained below.

Preferable examples of "organic group" represented by R¹ include alkyl, fluoroalkyl, alkoxycarbonyl, and aromatic groups.

More preferable examples of "organic group" represented by R¹ include fluoroalkyl.

Examples of "alkoxycarbonyl" include C₁₋₆ alkoxycarbonyl, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentabutoxycarbonyl, isopentoxycarbonyl, and hexyloxycarbonyl.

Preferable examples of "aromatic groups" include aryl, and more preferably, C₆₋₁₀ aryl, such as phenyl and naphthyl.

R^{X} is preferably fluorine.

When R^{2b} and R^{2c} join together to form a bond, as would be easily understood by a person skilled in the art, the structure of formula (1) is a structure represented by the following chemical formula: 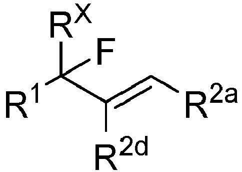 and the structure of formula (3) is a structure represented by the following chemical formula:

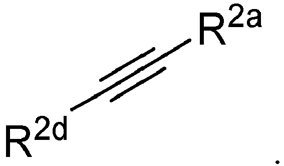

At least one of R^{2a}, R^{2b}, R^{2c}, or R^{2d} is preferably an electron-releasing group.

In a preferable embodiment of the present invention, at least one of R^{2a}, R^{2b}, R^{2c}, or R^{2d} can be hydrocarbon optionally having at least one substituent.

Examples of the substituent include heteroaryl optionally having at least one substituent, (more preferably 5-to 18-membered heteroaryl optionally having at least one substituent), thioether optionally having at least one substituent, and silazane optionally having at least one substituent.

Preferable examples of "substituent" in the "heteroaryl optionally having at least one substituent," "thioether optionally having at least one substituent," or "silazane optionally having at least one substituent" include halogen (preferably fluorine), cyano, amino, alkoxy, perfluoro organic groups (preferably C₁₋₈ perfluoro organic groups, more preferably trifluoromethyl), and pentafluorosulfanyl (F₅S-).

In a preferable embodiment of the present invention, at least one of R^{2a}, R^{2b}, R^{2c}, or R^{2d} can be unsubstituted hydrocarbon (preferably C₁₋₁₀ hydrocarbon) .

Preferable examples of "hydrocarbon" include alkyl (preferably C₁₋₁₀ alkyl) , cycloalkyl (preferably C₃₋₁₀ cycloalkyl, and more preferably C₄₋₈ cycloalkyl), and aryl (preferably C₆₋₁₀ aryl).

In a more preferable embodiment of the present invention, R^{2a} is alkyl or aryl, and R^{2b}, R^{2c}, and R^{2d} are each hydrogen.

Examples of the leaving group represented by X include halogen atoms (such as fluorine, chlorine, bromine, and iodine), alkylsulfonyloxy (C₁₋₆ alkylsulfonyloxy, such as methane sulfonyloxy and trifluoromethane sulfonyloxy), arylsulfonyloxy (C₆₋₁₀ arylsulfonyloxy, such as benzene sulfonyloxy and p-toluenesulfonyloxy).

Preferable examples of X include halogen atoms.

More preferable examples of X include chlorine, bromine, and iodine.

Even more preferable examples of X include bromine.

In a preferable embodiment of the present invention, R¹ is fluoroalkyl, alkoxycarbonyl, or aryl;
R^{X} is fluorine;
R^{2a}, R^{2b}, R^{2c}, and R^{2d} are the same or different, and each represents alkyl (preferably C₁₋₁₀ alkyl), cycloalkyl (preferably C₃₋₁₀ cycloalkyl, and more preferably C₄₋₈ cycloalkyl), or aryl (preferably C₆₋₁₀ aryl); and
X is bromine.

The amount of compound (3) in step A is preferably within 0.5 to 10 mol, more preferably within 1 to 8 mol, and even more preferably within 1.2 to 6 mol, per mol of compound (2).

When compound (3) has at least one carbon-carbon double bond in addition to the carbon-carbon double bond shown in the structural formula of formula (3), compound (1) can be a ring-closed derivative of the compound represented by formula (1) by ring closure reaction. The ring formed by the ring closure reaction is preferably a 5- to 7-membered ring. The ring formed by the ring closure reaction may be a carbocyclic ring, or a heterocyclic ring containing, in addition to carbon, at least one (preferably one or two) heteroatom selected from the group consisting of nitrogen, sulfur, and oxygen, as an annular atom.

When R^{2a} is epoxy (i.e., when compound (3) is an epoxy compound), compound (1) can be a ring-opened derivative (i.e., epoxy ring-opened derivative) of the compound represented by formula (1) by ring-opening reaction.

The reaction of step A is performed in the presence of a reducing agent.

The reducing agent used in the present invention may be an inorganic or organic reducing agent. Examples of the reducing agent include hydrogen, formic acid, ammonium formate, sodium formate, formic acid triethylamine, triethylsilan, tetramethyl disiloxane, polymethylhydrosiloxane, NaBH₃CN, NHCBH₃ (N-heterocyclic carbene boranes), and a nitrogen-containing unsaturated heterocyclic compound having an N-H moiety (imino group).

Preferable examples of the reducing agent used in the present invention include a nitrogen-containing unsaturated heterocyclic compound having an N-H moiety.

Examples of the "nitrogen-containing unsaturated heterocyclic compound having an N-H moiety" that can be used as a reducing agent in the present invention include a compound represented by formula (4) (in the specification, sometimes referred to as "compound (4)"), 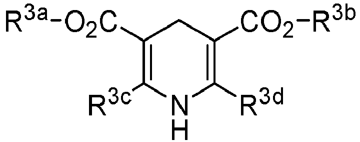 wherein R^{3a}, R^{3b}, R^{3c}, and R^{3d} are the same or different, and each represents alkyl.

R^{3a} is preferably C₁₋₆ alkyl, more preferably methyl or ethyl.

R^{3b} is preferably C₁₋₆ alkyl, more preferably methyl or ethyl.

R^{3c} is preferably C₁₋₆ alkyl, more preferably methyl or ethyl.

R^{3d} is preferably C₁₋₆ alkyl, more preferably methyl or ethyl.

More preferable examples of the reducing agent that can be used in the present invention include compounds represented by the formulae below. These compounds are "Hantzsch esters."

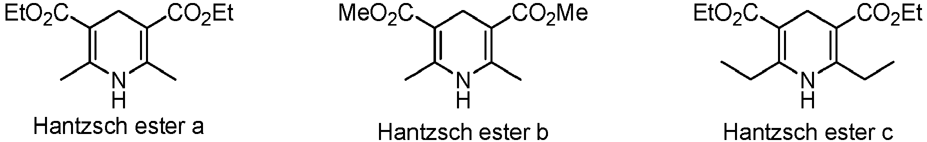

According to common technical knowledge in the field of organic chemistry, the following is considered. Since a halogenated alkane derivative containing compound (2) is easily oxidizable, a reducing agent such as a Hantzsch ester directly causes an oxidation-reduction reaction with the derivative, which prevents the reaction of step A, failing to obtain compound (1). However, surprisingly, the reaction of step A suitably proceeds in the presence of compound (4) containing a Hantzsch ester. The results are shown in the Examples.

Such reducing agents can be used alone or in a combination of two or more.

In the reaction of step A, an acid-removing agent, such as amine, can be optionally used.

When compound (4) is used, it is preferable not to use other amines.

When the reducing agent is used in step A, the amount of the reducing agent is preferably within 0.5 to 10 mol, more preferably 1.0 to 5.0 mol, and even more preferably 1.2 to 3.0 mol, per mol of the compound represented by formula (2), which is used as a substrate.

The reaction of step A can be performed in the presence of a catalyst or in the substantially or completely absence of a catalyst.

The reaction of step A is preferably performed in the presence of a catalyst.

Examples of the catalyst used in the present invention include transition metal complexes and organic dye compounds.

Examples of the kinds of central metal contained in the transition metal complexes that can be used in the present invention include cobalt, ruthenium, rhodium, rhenium, iridium, nickel, palladium, osmium, and platinum.

Preferable examples of the kinds of central metal include ruthenium, iridium, and palladium.

Examples of ligands of the transition metal complexes that can be used in the present invention include nitrogen-containing compounds, oxygen-containing compounds, and sulfur-containing compounds.

Examples of "nitrogen-containing compounds" used as ligands include diamine compounds (e.g., ethylenediamine) and nitrogen-containing heterocyclic compounds (e.g., pyridine, bipyridine, phenanthroline, pyrrole, indole, carbazole, imidazole, pyrazole, quinoline, isoquinoline, acridine, pyridazine, pyrimidine, pyrazine, phthalazine, quinazoline, and quinoxaline.)

Examples of "oxygen-containing compounds" used as ligands include diketones (e.g., dipivaloyl methane) and oxygen-containing heterocyclic compounds (e.g., furan, benzofuran, oxazol, pyran, pyrone, coumarin, and benzopyrone).

Examples of "sulfur-containing compounds" used as ligands include sulfur-containing heterocyclic compounds (e.g., thiophene, thionaphthene, and thiazole).

In the transition metal complex, the number of ligands of these compounds can be one or more. However, needless to say, the number of ligands may not necessarily be clear.

When a catalyst is used in the reaction of step A, the amount of the catalyst in step A is preferably within 0.0001 to 0.1 mol, more preferably 0.001 to 0.05 mol, and even more preferably 0.005 to 0.02 mol, per mol of compound (2).

The organic dye compound that can be used in the present invention can be a compound containing no metal atom in a molecule.

Examples of such organic dye compounds include rose bengal, erythrosine, eosine (e.g., eosine B and eosine Y), acriflavine, riboflavine, and thionine.

Preferable examples of catalysts include [Ir{dF(CF₃) ppy}₂ (dtbpy)]PF₆, [Ir(dtbbpy) (ppy)₂][PF₆], Ir (ppy) ₃, Ru (bpy) ₃Cl₂·6H₂O, [Ru (bpz) ₃] [PF₆]₂, [Ru (bpm) ₃] [Cl]₂, [Ru (bpy) ₂ (phen-5-NH₂)][PF₆]₂, [Ru (bpy) ₃] [PF₆] ₂, Ru (phen) ₃Cl₂, Cu(dap)₂ chloride, 9-mesityl-10-methyl acridinium·perchlorate, Ir(ppy)₃, and Pd(PPh₃)₄.

The catalysts can be used alone or in a combination of two or more.

A photoredox catalyst can be preferably used as the catalyst used in step A.

The catalyst used in step A may be carried by a carrier (e.g., zeolite).

The reaction of step A can be performed in the presence of a solvent or in the substantially or completely absence of a solvent.

The reaction of step A is preferably performed in the presence of a solvent.

Examples of solvents used in the present invention include dimethylformamide (DMF), toluene, CH₃CN, ether, tetrahydrofuran (THF), benzene, dimethylsulfoxide (DMSO), hexane, and benzotrifluoride (BTF).

These solvents can be used alone or in a combination of two or more.

When the reaction of step A starts, the concentration of compound (2) in the mixture of the reaction system is preferably within 1 to 10000 mM, more preferably within 10 to 1000 mM, and even more preferably within 50 to 200 mM.

When the reaction of step A starts, the concentration of compound (3) in the mixture of the reaction system is preferably within 5 to 50000 mM, more preferably within 50 to 5000 mM, and even more preferably within 250 to 1000 mM.

When the catalyst is used in the reaction of step A, the concentration of the catalyst in the mixture of the reaction system is preferably 0.01 to 100 mM, more preferably 0.1 to 10 mM, and even more preferably 0.5 to 2 mM.

Step A can be performed by mixing compound (2), compound (3), a desired reducing agent, a desired catalyst, and a desired solvent.

Conventional methods can be used for mixing these substances.

In the mixing, all of the substances can be simultaneously mixed, or sequentially or gradually mixed.

The reaction of step A is performed under light irradiation.

Any irradiation light can be used for light irradiation as long as light can start and/or promote the reaction of step A. Examples of the light source include a low-pressure, medium-pressure, or high-pressure mercury-vapor lamp, tungsten lamp, and light-emitting diode (LED).

The irradiation light can be preferably a visible light.

The irradiation light is preferably light having a wavelength of 300 to 600 nm, and more preferably light having a wavelength of 400 to 500 nm.

The irradiation time is preferably within 1 to 24 hours, and more preferably within 10 to 18 hours.

The light irradiation can start before, during, at the same time as, or after mixing.

The intensity of light irradiation may be such that energy for starting and/or promoting the reaction of step A is supplied. For example, the intensity of light irradiation can be suitably adjusted by adjusting, based on common technical knowledge, the output of the light source, the distance between the light source and the reaction system of step A, etc., so that the reaction of step A suitably proceeds.

The reaction of step A can be performed in the presence of an inert gas. Examples of the inert gas include nitrogen and argon.

The reaction temperature in step A is preferably within 0 to 120°C, more preferably within 10 to 80°C, and even more preferably within 20 to 60°C.

An excessively low reaction temperature may cause insufficient reaction of step A.

An excessively high reaction temperature is disadvantageous in view of costs and may cause undesirable reaction.

The reaction time in step A is preferably within 1 to 24 hours, more preferably within 5 to 18 hours, and even more preferably within 10 to 15 hours.

An excessively short reaction time may cause insufficient reaction of step A.

An excessively long reaction time is disadvantageous in view of costs and may cause undesirable reaction.

The reaction of step A can be preferably performed in a batch manner or in a flow system.

Compound (1) obtained by the production method of the present invention can be purified, as desired, by a known purification method, such as solvent extraction, drying, filtration, distillation, concentration, and a combination thereof.

According to the production method of the present invention, the inversion rate of compound (2), which is a starting material, is preferably 40% or more, more preferably 60% or more, and even more preferably 80% or more.

According to the production method of the present invention, the selectivity of compound (1) is preferably 70% or more, and more preferably 80% or more.

According to the production method of the present invention, the yield of compound (1) is preferably 40% or more, and more preferably 60% or more.

Compound (1) obtained by the production method of the present invention can be used, for example, as a pharmaceutical intermediate.

### Examples

The present invention is detailed below with reference to Examples; however, the present invention is not limited to these.

In the Examples below, yields are isolated yields unless otherwise specified.

### Example 1

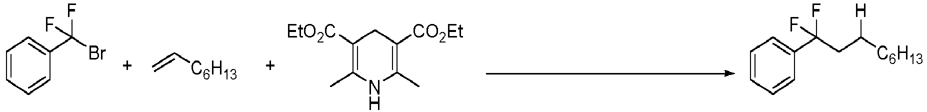

(1) Ru (bpy) ₃Cl₂·6H₂O (7.5 mg, 1 mol%) used as a photoredox catalyst, and Hantzsch ester a (380.7 mg, 1.5 mmol) were added to a container, and the mixture was dissolved in DMF (5 mL). (Bromodifluoromethyl)benzene (206.6 mg, 1.0 mmol), 1-octene (0.78 mL, 5.0 mmol), Et₃N (201.0 mg, 1.99 mmol), and DMF (5 mL) were then added to the mixture to perform Ar replacement, and the resultant was stirred under white-light irradiation for 12 hours.
   After the reaction, 40 mL of a solution containing EtOAc/hexane =9/1 was added to the solution, and the organic layer was washed with 20 mL of pure water 3 times and 30 mL of saturated saline once. Thereafter, the organic layer was dehydrated, filtered, and dried, and then silica gel column chromatography (developing solvent: hexane) was performed to obtain 1,1-difluoro-1-phenylnonane (151.6 mg, yield: 63%).
(2) The same reaction as in Item (1) above was performed except that a photoredox catalyst was not used.
(3) The same reaction as in Item (1) above was performed except that Et₃N was not used.

The results are shown in the table below. As understood from the results, the conversion rate was improved when the photoredox catalyst was used. The reaction suitably proceeded regardless of the presence or absence of Et₃N.

**Table 1**

| | Ru (bpy) ₃Cl₂·6H₂O | Et₃N (equiv) | conv. (%) ^{a} | yield (%) |
|---|---|---|---|---|
| (1) | 1 | 2 | 100 | 63 |
| (2) | 0 | 2 | 39 | 32 ^{b} |
| (3) | 1 | 0 | 96 | 63 ^{b} |

| | | | | |
|---|---|---|---|---|
| ^{a}: Using undecane as an internal standard, the conversion rate was determined by GC. ^{b}: NMR yield | | | | |

(4) Using compounds (2) and (3) in the table below, compounds (1) shown in the table below were obtained in the same manner as in Item (1) above. The results are shown in the table below together with the results of Item (1) above.

**Table 2**

| Compound (2) | Compound (3) | Compound (1) (target compound) | Yield (compound(1)/other compound) ^{a} |
|---|---|---|---|
| 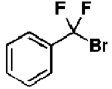 | 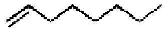 | 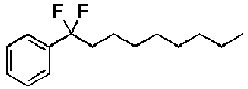 | 63% (96/4) |
| 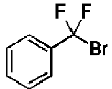 | 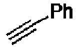 | 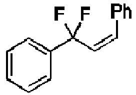 | 35% (E/Z = 15/85) with Et₃N |
| | | | 79% (E/Z = 11/89) w/o Et₃N |
| 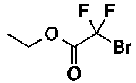 | 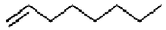 | 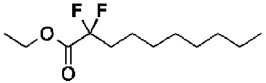 | 60% (89/11) |
| 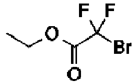 | 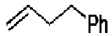 | 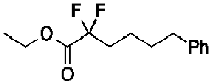 | 55% (94/6) |
| 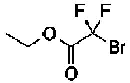 | 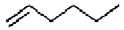 | 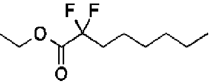 | 64% (87/13) |
| 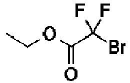 | 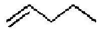 | 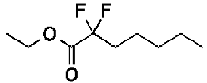 | 61 % (86/14) |
| 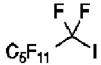 | 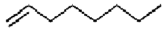 | 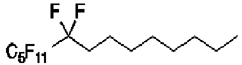 | 56% (92/8) |
| 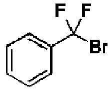 | 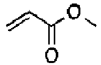 | 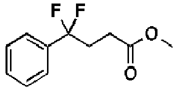 | 71% |
| 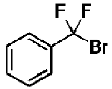 | 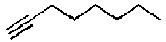 | 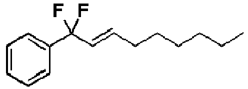 | 29% (E/Z = 41/59) |
| 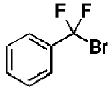 | 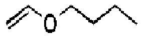 | 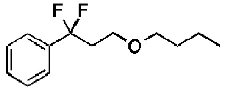 | complex mixture |
| 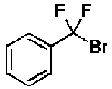 | 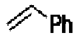 | 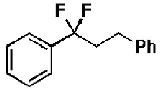 | 6%, conv = 48% (12 h) |
| | | | 10%, conv = 99% (70 h) |
| 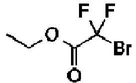 | 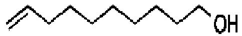 | 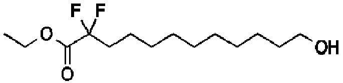 | 49% |
| 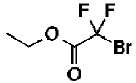 | 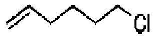 | 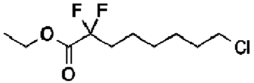 | 51% |
| 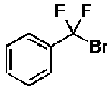 | 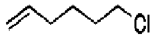 | 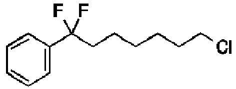 | 61% |

| | | | |
|---|---|---|---|
| ^{a}: The rate of compound (1)/other compound is based on the isolate yield. | | | |

(5) In some starting material compounds (table below), in addition to target compound a (Compound (1)), bromine atom - transferred compound b, reductive adduct c in which two molecules of olefin were involved, and a bromine atom mobile in which two molecules of olefin were involved were simultaneously obtained by reactions (1) to (4). Regarding each starting material, the table below shows the yield, and the GC area ratio of compounds a, b, c, and d. 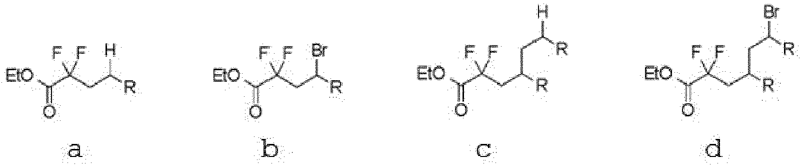 (R in each formula corresponds to R^{2a}.)

**Table 3**

| Yield (GC area ratio of a:b:c:d) | | |
|---|---|---|
| 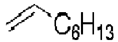 | 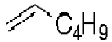 | 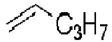 |
| 60% (89 : 3 : 3 : 4) | 64% (87 : 4 : 4 : 5) | 61% (86 : 5 : 3 : 6) |
| 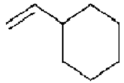 | | 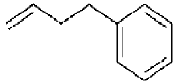 |
| 46% (94 : 3 : 2 : 1) | | 55% (94 : <1 : 2 : 4) |

### Example 2

In the same manner as in Example 1 (1), the reaction of Example 1 (1) was performed under conditions shown in the table below. The results are shown in the table below.

**Table 4**

| | | | | | |
|---|---|---|---|---|---|
| 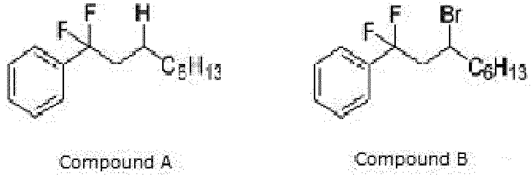 | | | | | |

| Solvent | Reducing agent | Catalyst | Conversion rate (%)^{a} | GC yield (%) | |
|---|---|---|---|---|---|
| 10 mL | 1.5 equiv | 1 mol % | | Compound A | Compound B |
| CH₃CN | Hantzsch ester a | Ru (bpy) ₃Cl₂·6H₂O | 100 | 70 | 12 |
| CH₂Cl₂ | Hantzsch ester a | Ru (bpy) ₃Cl₂·6H₂O | 93 | 60 | 7 |
| MeOH | Hantzsch ester a | Ru (bpy) ₃Cl₂·6H₂O | 88 | 60 | 6 |
| DMF | Hantzsch ester a | Ru (bpy) ₃Cl₂·6H₂O | 100 | 86 | 4 |
| DMF | Hantzsch ester b | Ru (bpy) ₃Cl₂·6H₂O | 100 | 61 | 6 |
| DMF | Hantzsch ester c | Ru (bpy) ₃Cl₂·6H₂O | 100 | 68 | 3 |
| DMF | Hantzsch ester a | Ru (bpy) ₃Cl₂·6H₂O | 100 | 86 | 4 |
| DMF | Hantzsch ester a | - | 39 | 34 | 3 |
| DMF | Hantzsch ester a | Ir (ppy)₃ | 100 | 53 | 4 |
| DMF | Hantzsch ester a | Rose bengal | 35 | 31 | 3 |
| DMF | - | Ru (bpy) ₃Cl₂·6H₂O | 55 | 21 | 25 |
| DMF | - | Eosin Y | 29 | 10 | 15 |
| DMF | - | Rose bengal | 42 | 5 | 6 |

| | | | | | |
|---|---|---|---|---|---|
| ^{a}: Using undecane as an internal standard, the conversion rate was determined by GC. | | | | | |

### Example 3

In the same manner as in Example 1 (1), (bromodifluoromethyl)benzene (1.0 mmol), 1-octene (5.0 mmol), a photoredox catalyst shown in the table below (1 mol% or 0 mol% relative to (bromodifluoromethyl)benzene), Hantzsch ester a (1.5 mmol) , Et₃N (2.0 mmol), and DMF (10 mL) were added to a container to perform Ar replacement. Thereafter, the resultant was stirred for 12 hours under light irradiation using a light source shown in the table below. White LED (5W) was used as a white light. A SOLARBOX 1500e (xenon lamp, soda-lime glass UV filter, produced by CO.FO.ME.GRA. Srl) was used as a daylight color light.

After the reaction, 40 mL of a solution containing EtOAc/hexane = 9/1 was added to the solution, and the organic layer was washed with 20 mL of pure water 3 times, and 30 mL of saturated saline once. Thereafter, the organic layer was dehydrated, filtered, and dried, and then silica gel column chromatography (developing solvent: hexane) was performed to obtain 1,1-difluoro-1-phenylnonane.

The conversion rate and GC yield are shown in the table below.

**Table 5**

| | | | | |
|---|---|---|---|---|
| 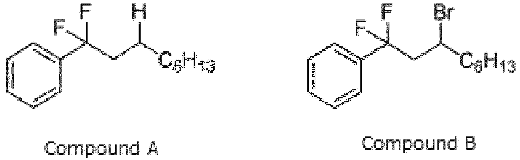 | | | | |

| Photoredox catalyst | Light source | Conversion rate (%)^{a} | GC yield (%) | |
|---|---|---|---|---|
| 1 mol% | | | Compound A | Compound B |
| - | White light | 39 | 34 | 3 |
| Ru (bpy) ₃Cl₂ ▪ 6H₂O | White light | 100 | 86 | 4 |
| Pd (PPh₃) ₄ | White light | 56 | 37 | 3 |
| Pd (PPh₃) ₄ | Daylight color light | 100 | 78 | 4 |
| - | Daylight color light | 94 | 70 | 4 |

| | | | | |
|---|---|---|---|---|
| ^{a}: Using undecane as an internal standard, the conversion rate was determined by GC. | | | | |

### Example 4

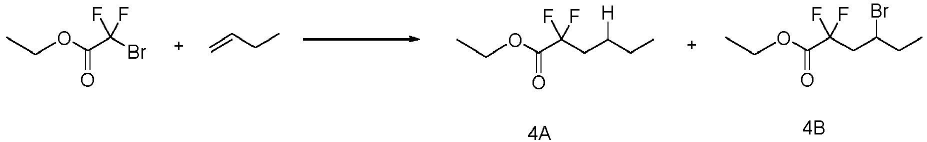

In the same manner as in Example 1 (1), ethyl 2-bromo-2,2-difluoroacetate (1.0 mmol), 1-butene (amount in the table below), and Ru(bpy)₃Cl₂·6H₂O (1.0 mol% relative to ethyl 2-bromo-2,2-difluoroacetate), Hantzsch ester a (1.5 mmol), Et₃N (1.0 mmol), and DMF (5 mL) were added to a container to perform Ar replacement. The resultant was then stirred under white-light irradiation for 12 hours.

After the reaction, the solution was purified in the same manner as in Example 1 (1) to obtain compound 4A.

The yield and GC yield are shown in the table below.

**Table 6**

| 1-Butene | Yield | GC yield (4A/4B) |
|---|---|---|
| 5.0 mmol | 44% | 94%/ 6% |
| 10.0 mmol | 35% | 94%/ 6% |

### Example 5

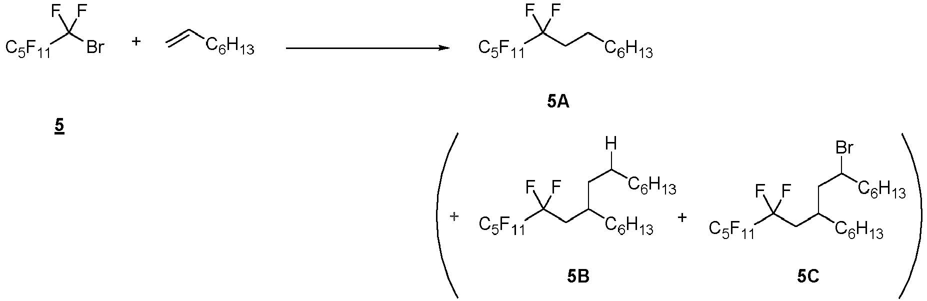

In the same manner as in Example 1 (1), perfluorohexylbromide 5 (1.0 mmol), 1-octene (amount in the table below), and Ru (bpy) ₃Cl₂·6H₂O (1 mol% relative to perfluorohexylbromide 5), Hantzsch ester a (1.5 mmol), Et₃N (1.0 mmol), and DMF (5 mL) were added to a container to perform Ar replacement. The resultant was stirred under white-light irradiation for 12 hours.

The reaction solution was purified in the same manner as in Example 1 (1) to obtain compound 5A.

The yield and GC yield are shown in the table below.

As shown in the table below, when the amount of 1-octene was increased from 5 mol equivalents to 20 mol equivalents, the yield of compound 5A was improved; however, by-products (compound 5B, compound 5C) to which two molecules of 1-octene were added were simultaneously generated, reducing the selectivity of compound 5A.

**Table 7**

| 1-Octene | Yield | GC Yield (5A/5B/5C) |
|---|---|---|
| 5.0 mmol | 50% | 92%/ 5%/ 3% |
| 20.0 mmol | 78% | 76%/11%/13% |

### Example 6

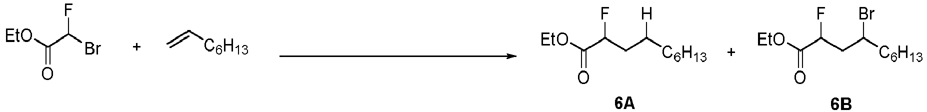

In the same manner as in Example 1 (1), ethyl bromofluoroacetate (1.0 mmol), 1-octene (5 mol equivalents, 10 mol equivalents, or 20 mol equivalents relative to ethyl bromofluoroacetate), Ru(bpy)₃Cl₂·6H₂O (1 mol% relative to ethyl bromofluoroacetate), Hantzsch ester a (1.5 mmol), Et₃N (1.0 mmol), and DMF (5 mL) were added to a container to perform Ar replacement. The resultant was stirred under white-light irradiation for 12 hours.

The reaction solution was purified in the same manner as in Example 1 (1) to obtain compound 6A.

The yield and GC yield are shown in the table below.

As shown in the table, when the amount of 1-octene was increased from 5 mol equivalents to 20 mol equivalents, the yield of compound 6A was improved.

**Table 8**

| 1-Octene (equiv) | NMR yield | GC yield (6A/6B) |
|---|---|---|
| 5 | 47% | 90/10 |
| 10 | 49% | 90/10 |
| 20 | 80% | 90/10 |

### Example 7

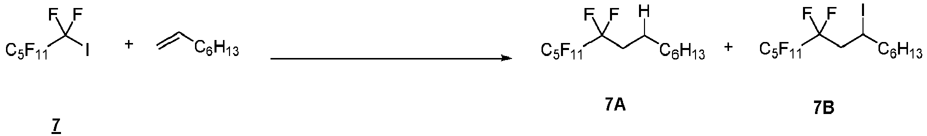

In the same manner as in Example 1 (1), perfluorohexyl iodide 7 (1.0 mmol) , 1-octene (5.0 mmol) , Ru(bpy)₃Cl₂.6H₂O (1.0 mol% relative to perfluorohexyl iodide 7), Hantzsch ester a (1.5 mmol), Et₃N (0 or 2.0 mmol), and DMF (5 to 10 mL) were added to a container to perform Ar replacement. The resultant was then stirred under white-light irradiation for 12 hours.

After the reaction, the solution was purified in the same manner as in Example 1 (1) to obtain compound 7A.

The conversion rate in each case was 100%.

The yield and GC yield are shown in the table below.

As shown in the table, compound 7A was obtained with an excellent yield regardless of the presence or absence of triethylamine.

**Table 9**

| Et₃N | Yield | GC Yield (7A/7B) |
|---|---|---|
| 2.0 mmol | 45% | 56% / 4% |
| 0.0 mmol | 56% | 45% /40% |

### Example 8

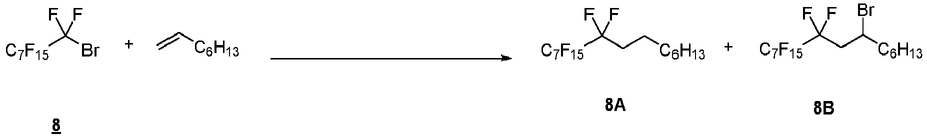

In the same manner as in Example 1 (1), perfluorooctylbromide 8 (1.0 mmol), Ru (bpy) ₃Cl₂·6H₂O (1.0 mol% relative to perfluorooctylbromide 8), 1-octene (20 mmol), Hantzsch ester a (1.5 mmol), Et₃N (0 or 2.0 mmol), and DMF (5 to 10 mL) were added to a container to perform Ar replacement. The resultant was then stirred under white-light irradiation for 12 hours.

After the reaction, the solution was purified in the same manner as in Example 1 (1) to obtain fluorinated compound 8A.

The yield is shown in the table below.

**Table 10**

| Et₃N | Yield (8A/8B) |
|---|---|
| 2.0 mmol | 90%/ 0% |
| 0.0 mmol | 86%/ 0% |

### Example 9

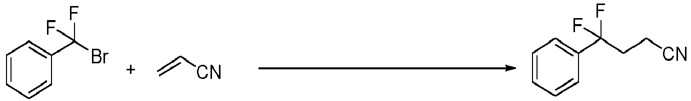

In the same manner as in Example 1 (1), (bromodifluoromethyl)benzene (1.0 mmol), acrylonitrile (5.0 mmol), Ru (bpy) ₃Cl₂·6H₂O (1.0 mol% to (bromodifluoromethyl) benzene), Hantzsch ester a (1.5 mmol), Et₃N (1.0 mmol), and DMF (5 mL) were added to a container to perform Ar replacement. The resultant was then stirred under white-light irradiation for 12 hours.

After the reaction, the solution was purified in the same manner as in Example 1 (1) to obtain compound 8A.

The yield was 77%.

### Example 10 (Reaction 1 in the flow system)

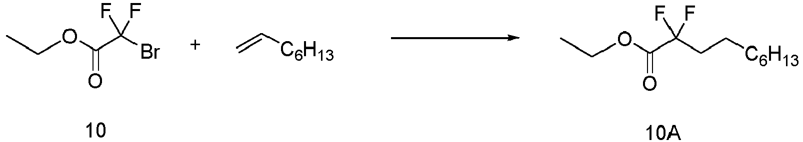

Ethyl 2-bromo-2,2-difluoroacetate (1.0 mmol) was reacted with 1-octene (5.0 mmol) in the flow system in the presence of Ru(bpy)₃Cl₂·6H₂O (1.0 mol% to ethyl 2-bromo-2,2-difluoroacetate), Hantzsch ester a (1.5 mmol), Et₃N (2.0 mmol), and DMF (10 ml) using an optical microreactor (white-light (white-LED) irradiation) having a flow channel (width: 1 mm, depth: 300 µm, and length: 2.35 m) .

As a result, compound 10A was obtained at a yield of 56% in a residence time of 30 minutes.

### Example 11 (Reaction 2 in the flow system)

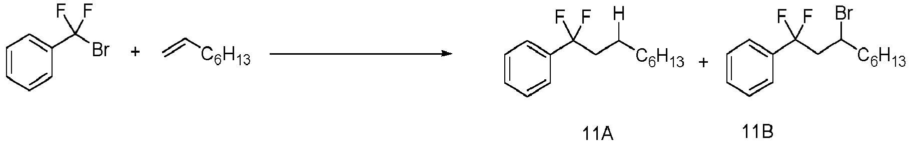

(Bromodifluoromethyl)benzene (1.5 mmol) was reacted with 1-octene (7.5 mmol) in the flow system in the presence of Ru (bpy) ₃Cl₂·6H₂O (1.0 mol% to (bromodifluoromethyl)benzene), Hantzsch ester a (1.5 mol equivalents (2.25 mmol) to (bromodifluoromethyl)benzene)), and DMF (15 mL), using an optical microreactor (white-light irradiation) having a flow channel (width: 2 mm, depth: 1 mm, and length: 3m).

As a result, compound 10A was obtained at a yield of 56% in a residence time of 30 minutes.

The conversion rate and GC yield for each residence time are shown in the table below.

**Table 11**

| Residence time (min) | Conversion rate | GC yield (11A/11B) |
|---|---|---|
| 30 | 83% | 48% / 3% |
| 40 | 85% | 56% / 4% |
| 50 | 89% | 61% / 4% |

As shown in the table, the yield was improved as the residence time became longer. As a result of the same reaction performed for 12 hours, the GC yield of compound 11A was 86% (yield: 64%). In view of the reaction time, it was confirmed that the flow system reaction produced a target object more efficiently than the batch reaction.

### Example 12 (Reaction 3 in the flow system)

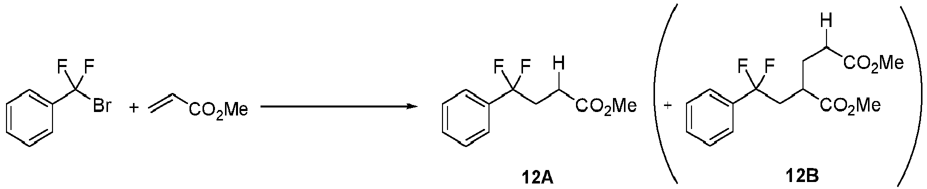

The reaction in the flow system was performed in the same manner as in Example 11 except that methyl acrylate was used in place of 1-octene as a substrate.

The results are shown in the table below.

As a result of the same reaction performed for 12 hours, the GC yield of compound 12A was 90% (yield: 72%). In view of the reaction time, it was confirmed that the flow system reaction produced a target object more efficiently than the batch reaction.

**Table 12**

| Residence time (min) | Conversion rate | GC yield (12A/12B) |
|---|---|---|
| 50 | 100% | 87% / 9% |
| 40 | 100% | 89% / 12% |
| 30 | 100% | 80% / 11% |
| 20 | 100% | 82% / 12% |
| 10 | 71% | 55% / 6% |

### List of embodiments

### [Item 1]

A method for producing a compound represented by the following formula (1) or a ring-closed or ring-opened derivative of the compound: 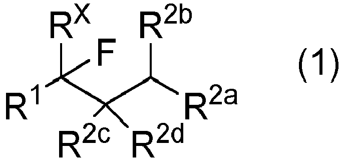 wherein R¹ represents an organic group,
R^{X} represents hydrogen or fluorine,
R^{2a}, R^{2b}, R^{2c}, and R^{2d} are the same or different, and each represents -Y-R²¹ or -N(-R²²)₂, or R^{2b} and R^{2c} may join together to form a bond,
wherein Y represents a bond, oxygen, or sulfur,
R²¹ represents hydrogen or an organic group, and
R²², in each occurrence, is the same or different and represents hydrogen or an organic group;
the method comprising step A of reacting a compound represented by formula (2): 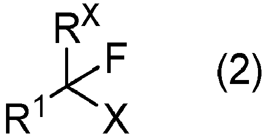 wherein X represents a leaving group, and other symbols are as defined above, with
a compound represented by formula (3): 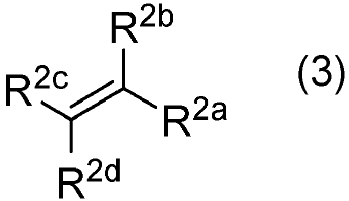 wherein the symbols are as defined above,
in the presence of a reducing agent under light irradiation.

### [Item 2]

The method according to item 1, wherein R¹ is alkyl, fluoroalkyl, alkoxycarbonyl, or an aromatic group.

### [Item 3]

The method according to item 1 or 2, wherein R^{2a} is alkyl or aryl, and R^{2b}, R^{2c}, and R^{2d} are each hydrogen.

### [Item 4]

The method according to any one of items 1 to 3, wherein X is bromine.

### [Item 5]

The method according to any one of items 1 to 4, wherein the reaction in step A is performed in the presence of a nitrogen-containing unsaturated heterocyclic compound having an N-H moiety.

### [Item 6]

The method according to any one of items 1 to 5, wherein the reducing agent is a compound represented by formula (4) : 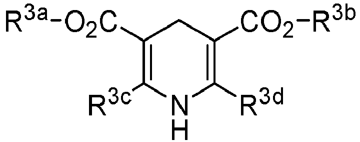 wherein R^{3a}, R^{3b}, R^{3c}, and R^{3d} are the same or different, and each represents alkyl.

### [Item 7]

The method according to any one of items 1 to 6, wherein the reaction in step A is performed in the presence of a catalyst.

### [Item 8]

The method according to item 7, wherein the catalyst is at least one member selected from the group consisting of transition metal complexes and organic dye compounds.

## Claims

1. A method for producing a compound of formula (1) or a ring-closed or ring-opened derivative of the compound: 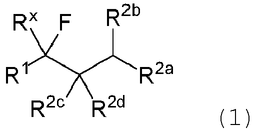 wherein
R¹ is an organic group,
R^{X} is H or F,
R^{2a}-R^{2d} each independently are -Y-R²¹ or -N (-R²²) ₂, and R^{2b} and R^{2c} may join together to form a bond, wherein Y is a bond, O or S,
R²¹ is H or an organic group, and
R²² each independently is H or an organic group;
the method comprises the step (A) of reacting, in a flow system, a compound of formula (2): 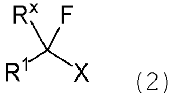 wherein R¹ and R^{X} are as defined above, and X is a leaving group, with a compound of formula (3): 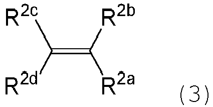 wherein R^{2a}-R^{2d} are as defined above, in the presence of a reducing agent under light irradiation.

2. The method of claim 1, wherein R¹ is alkyl, fluoroalkyl, alkoxycarbonyl or an aromatic group.

3. The method of claim 1 or 2, wherein R^{2a} is alkyl or aryl, and R^{2b}, R^{2c} and R^{2d} are each H.

4. The method of any of claims 1-3, wherein X is Br.

5. The method of any of claims 1-4, wherein step (A) is performed in the presence of a nitrogen-containing unsaturated heterocyclic compound having a N-H moiety.

6. The method of any of claims 1-5, wherein the reducing agent is a compound of formula (4) wherein R^{3a}-R^{3d} each independently are alkyl:

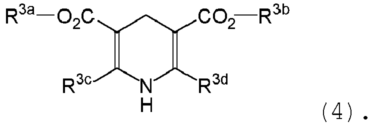

7. The method of any of claims 1-6, wherein step (A) is performed in the presence of a catalyst.

8. The method of claim 7, wherein the catalyst is at least one of transition metal complexes and organic dye compounds.
